# EUROPEAN PATENT APPLICATION

(11) **EP 2 676 727 A1**
(43) Date of publication of application: **25.12.2013**
(21) Application number: 12172594.9
(22) Date of filing: 19.06.2012
(51) Int. Cl.: B01J 20/282, C07K 14/47, G01N 33/68

(54) **Method for the capture or removal of amyloidogenic proteins**

(71) Applicant: Prionatis AG, 6055 Alpnach Dorf (CH)
(72) Inventor: Ruinatscha, Reto, 8005 Zürich (CH); Hagen, Alexander, 6025 Neudorf (CH)
(74) Representative: Heubeck, Christian

(57) **Abstract**

The present invention relates to methods for isolating, removing, concentrating, purifying and/or detecting amyloids, amyloidogenic proteins, fragments and/or derivatives thereof, and in particular to a method for the selective capture of amyloids, such as PrP^{Sc}. Another aspect of the invention is directed to devices for carrying out these inventive methods.

## Description

The present invention relates to methods for isolating, removing, concentrating, purifying and/or detecting amyloids, fragments and/or derivatives thereof, and in particular to a method for the selective capture of amyloidogenic proteins, such as PrP^{Sc}. Another aspect of the invention is directed to devices for carrying out these inventive methods.

Amyloids are fibrillar aggregates formed by normally soluble, innocuous proteins or peptides, and are characterized by a cross-β sheet quaternary structure. Diseases featuring amyloids include, but are not limited to, Alzheimer's disease, Diabetes mellitus type 2, Parkinson's disease, Huntington's Disease, and transmissible spongiform encephalopathies. (See, e.g., Chiti & Dobson (2006) Ann. Rev. Biochem. 75: 333-366; Westermark (2005) FEBS J. 272: 5942-5949; Ross & Poirier (2004) Nature Med.10: S10-S17; Dobson (2002) Nature 418: 729-730).

Transmissible spongiform encephalopathies (TSEs) are neurodegenerative diseases. They are largely untreatable and fatal. (See, e.g., Grillberger et al (2009) Biotechnol. J. 4: 186-201). TSEs include bovine spongiform encephalopathy (BSE) in cattle, chronic wasting disease (CWD) in deer and elk, scrapie in sheep and goats, as well as Creutzfeldt-Jakob disease (CJD), Gerstmann-Straussler-Scheinker (GSS) syndrome, fatal familial insomnia (FFI), and Kuru in humans. (See, e.g., Collinge (1999) Lancet 354: 317-323).

TSEs are associated with a conformational conversion of the cellular prion protein PrP^{C} into the pathogenic, β-sheet-rich prion protein PrP^{Sc}. PrP^{Sc} aggregations form highly structured amyloid fibers, which accumulate to form amyloid plaques in affected brains. (See, e.g., Kelly (1996) Curr. Opin. Struct. Biol. 6: 11-17; Pan et al (1993) P. Natl. Acad. Sci. USA 90(23): 10962-10966). Pathological prion forms may have various abbreviations that emphasize associations with infectivity (PrP^{Sc}), disease (e.g. PrP^{TSE} and PrP^{d}), toxicity (PrP^{tox} and PrP^{L}), and relative protease-resistance (PrPres).

TSEs, or prion diseases, can arise spontaneously, e.g. sporadic Creutzfeldt-Jakob disease (sCJD), through genetic predisposition, e.g. familial CJD, or through contaminated instruments, organ and tissue grafts, or oral transmission, e.g. variant CJD (vCJD). (See, e.g., Prusiner (2004) Prion Biology and Diseases, Cold Spring Harbor Lab Press, Woodbury, NY, 2nd Ed.).

Current findings strongly indicate that vCJD may also be transmitted via blood transfusions. (See, e.g., McCutcheon et al (2011) PLoS One 6(8): e23169; Chohan et al (2010) Transfusion 50: 1003-1006; Gillies et al (2009) Vox Sang. 97: 211-218; Ward et al (2009) Vox Sang. 97: 207-210; Health Protection Agency (2007) Fourth case of transfusion-associated variant-CJD infection. Health Protection Report 1 (3); Health Protection Agency (2006) New case of transfusion-associated variant-CJD. CDR Weekly 16(6); Hewitt PE ot al (2006) Vox Sang. 91: 221-230; Wroe et al (2006) Lancet 368: 2061-2067; Llewelyn et al (2004) Lancet 363: 417-421; Peden et al (2004) Lancet 364: 527-529). This risk also exists with blood derived therapeutic formulations, and potentially with all therapeutics and cosmetics which contain components of human or bovine source.

In addition, studies suggest that urine of prion-infected individuals may be infectious before the appearance of clinical signs, i.e. even before pathogenic prions are detectable by current methods in the urine, and that administration of urine-derived products could expose patients to a potential risk of prion transmission. (See, e.g., Dorsselaer et al (2011) PLoS One 6 (3): e17815).

In principle, all amyloid diseases could be infectious under certain conditions. (See, e.g., Sigurdsson (2002) Trends Mol. Med. 8(9): 411-413). For example, in Alzheimer's disease there is emerging evidence that aggregates of amyloid-β may spread from brain region to brain region and from cell to cell in a prion-like fashion. (See, e.g., Holtzman et al (2011) Sci. Transl. Med. 3(77): 77sr1; Kim & Holtzman (2010) Science 330: 918-919). An overview of prions and potential prion-like proteins is provided in the following table (adapted from: Aguzzi (2009) Nature 459(7249): 924-925):

| **Disease** | **Protein** | **Molecular transmissibility** | **Infectious life cycle** |
|---|---|---|---|
| Prion diseases | PrP^{Sc} | Yes | Yes |
| Alzheimer's disease | Amyloid-β | Yes | Not shown |
| Tauopathies | Tau | Yes | Not shown |
| Parkinson's disease | α-Synuclein | Host-to-graft | Not shown |
| AA amyloidosis | Amyloid A | Yes | Possible |
| Huntington's disease | Polyglutamine | Yes | Not shown |

In summary there is a strong need to minimize the potential risk of infection with amyloid diseases, such as prion diseases, most preferably at an early stage of these diseases, as this would significantly advance the chances of success of any possible intervention approach.

However, the detection of pathogenic prions, i.e. before the appearance of clinical signs, has proven to be very challenging due to several reasons. For example, easily accessible body fluids such as plasma contain only very low concentrations of the pathogenic prion protein PrP^{Sc}. The total protein concentration in human plasma is in the range of 60 - 85 mg/ml. In contrast, PrP^{C} concentrations are only around 10 ng/ml, and PrP^{Sc} concentrations of pre-symptomatic CJD patients are assumed to be as low as 30 fg/ml. Such PrP^{Sc} concentrations are by far below the detection limit of established analysis methods such as e.g. Western Blot (detection limit around 30'000 fg/ml) or enzyme-linked immunosorbent assay (ELISA; detection limit around 5'000 fg/ml).

Various methods for the diagnosis of amyloid diseases have been proposed over the past years, whereas on the example of prion diagnostic the following challenges and hurdles have to be addressed:
1. Target protein in protein rich, complex solutions such as e.g. blood plasma
2. Low concentration of the target proteins below the detection limits of classic immunological analysis methods
3. Pathogenic form (PrP^{Sc}) in much lower concentration than the normal form (PrP^{c})
4. Special efforts required to distinguish the pathogenic from the normal form
5. Overcome practical limitations

As of today only post-mortem prion tests have been approved by the European health authorities and no in vitro diagnostic test yet. To specifically address challenges (1) to (5) of above listing several methods are known from the prior art.

As already the detection of amyloidogenic proteins and in particular of pathogenic forms, e.g. PrP^{Sc}, turns out to be difficult, it is clear to the skilled person that methods for isolation, removal and/or concentration of corresponding amyloidogenic proteins are difficult to realize or are even not available.

It is therefore one object of the present invention to provide a method for the isolation, removal, concentration and/or purification of amyloids, amyloidogenic proteins and/or proteins with the ability to become amyloidogenic, and prion proteins in particular, that do not have the disadvantages of the methods known from the prior art. Moreover, it should be possible with the method of the present invention to enable reliable in vitro diagnosis of amyloid diseases, and prion diseases in particular, in an easily applicable manner. According to another object, devices for carrying out these processes are provided.

The problem underlying the present invention is solved by providing a method for the selective enrichment, isolation, removal, concentration or even separation of amyloids, amyloidogenic proteins and/or proteins with the ability to become amyloidogenic, in particular prion proteins (e.g. PrP^{C} and/or PrP^{Sc}, and/or PrPres), by contacting a metal matrix having a surface to volume ratio between 1 cm²/cm³ and 700 cm²/cm³ from protein-abundant liquids like blood plasma.

Thus, according to a first aspect the present invention relates to a method for isolating, removing, concentrating and/or purifying amyloids, amyloidogenic proteins and/or proteins with the ability to become amyloidogenic, fragments and/or derivatives thereof comprising a step (a) contacting a metal matrix having a surface to volume ratio between 1 cm²/cm³ and 700 cm²/cm³, in particular 1 cm²/cm³ and 500 m²/cm³, with a target fluid under conditions allowing for adsorption and/or capture of possibly present amyloids, amyloidogenic proteins and/or proteins with the ability to become amyloidogenic, fragments and/or derivatives thereof, wherein at least one detergent may be present during the contacting step (a). The selective enrichment, isolation, removal, concentration, separation or detection of amyloidogenic proteins is especially preferred.

The resulting solution has a substantially lower concentration of amyloids, amyloidogenic proteins and/or proteins with the ability to become amyloidogenic, fragments and/or derivatives thereof. In a particular preferred embodiment the concentration of amyloids, amyloidogenic proteins and/or fragments thereof is lowered. By the method according to the invention, the concentration of amyloidogenic proteins and/or fragments thereof can be reduced by a factor of 10 or more. In a preferred embodiment, the amyloids and/or amyloidogenic proteins are completely removed, of course. However, in other embodiments, such as for diagnostic purposes, it could already be sufficient if 50% of the pathogenic prions and/or amyloidogenic proteins are captured or concentrated. Thus, the reduction factor is particularly dependent from the purpose of the use and can thus, as outlined, differ in the production process or the diagnostic application.

In detail, according to the present invention it was found that amyloidogenic proteins, in particular pathogenic prions, contaminating a given sample such as a protein-containing solution are substantially removed by metal matrices even in the presence of large excess of other proteins, i.e. at high protein concentrations, e.g. in blood plasma. Most surprisingly, to achieve a more selective capture of PrP^{Sc}, the PrP^{C} capture yield can be reduced with the presence of detergent, such as surface pretreatment/coating of a metal matrix prior to contact with prions. Of course, detergent may also be added during contacting step (a).

Thus, according to a preferred embodiment, the inventive method can be used to separate and/or isolate etc. non-pathogenic prion proteins from pathogenic prions and/or amyloidogenic proteins, such as PrP^{Sc} from PrP^{C}.

According to the present invention, the terms "amyloid proteins" or "amyloids", which can be used interchangeably herein, designate insoluble fibrous proteins aggregates sharing specific structural traits. The accumulation and/or abnormal deposition of such "amyloid proteins" leads to amyloidosis and/or disease conditions. According to the present application, the terms "amyloid proteins" or "amyloids" relate to a native and/or denatured protein form. Thus, according to the invention amyloid proteins can be isolated, removed, concentrated, detected, purified and/or provided in a native and/or denatured form.

According to the present invention, the term "amyloidogenic protein" designates a potential precursor and/or intermediate protein of an amyloid.

Preferably, the amyloidogenic and/or amyloid proteins to be isolated, removed, concentrated and/or detected etc. are selected from beta amyloid (Aβ), which is, e.g., known from Alzheimer's disease, IAPP amylin (AIAPP), which is known, e.g., from diabetes mellitus type II, alpha-synuclein (SNCA), which is known from Parkinson's disease, Huntingtin, which is known from Huntington's disease and in particular prion proteins (PrP), which are, e.g., known from prion diseases like BSE, CJD, CWD, and scrapie. Prion proteins represent an especially preferred embodiment of the present invention and comprise in particular pathogenic PrP^{Sc}, non-pathogenic PrP^{C}, PrPres and/or recombinant prion proteins, fragments and/or derivatives thereof.

According to the invention, "fragment and/or derivative" relates to any functionally active molecule showing characteristics corresponding to the characteristics of the corresponding "wild-type full-length amyloid protein", such as biologically activity and/or antigenic properties. For example, in terms of the invention, a fragment of a prion protein shows antigenic properties corresponding to the prion protein itself and/or essentially the same biological activity.

The amyloid-, in particular PrP^{Sc} capture/removal yield using metal matrices was found to be dependent on the provided volumetric metal surface area or surface to volume ratio of the metal matrix. Due to different capabilities in capturing and affinities, i.e. of detergent pretreated/coated metal matrices, pathogenic and non-pathogenic prions can be separated according to the invention as already stated above. Of course, it is also contemplated by the present invention that in one embodiment amyloids, amyloidogenic proteins and/or proteins with the ability to become amyloidogenic, such as non-pathogenic prions and pathogenic prions can be captured using the metal matrix simultaneously.

According to the invention, the metal matrix has a surface to volume ratio between 1 cm²/cm³ and 700 cm²/cm³, preferably between 5 cm²/cm³ and 700 cm²/cm³, more preferably between 10 cm²/cm³ and 600 cm²/cm³, and even more preferably between 10 cm²/cm³ and 500 cm²/cm³ and most preferably between 15 cm²/cm³ and 500 cm²/cm³. The surface to volume ratio of the metal matrix can be calculated geometrically from the amount of surface area per unit volume of the metallic object or collection of objects, and may be measured using, e.g., multipoint BET from krypton adsorption. (See, e.g., Brunauer S et al (1938) J. Am. Chem. Soc. 60(2): 309-319).

Of course, the person skilled in the art can determine a suitable metallic material providing a metal matrix suitable to be used according to the present invention. A suitable test to determine whether a metal or matrix is suitable is described in the examples. In a preferred embodiment, the process according to the invention comprises a respective step, i.e. a step for determining whether a given metal or given metal matrix is suitable for the method according to the invention. However, the metal matrix used is preferably selected from metal-based materials comprising ferrous material, steel (unalloyed, low-alloyed, high-alloyed), cast iron, nonferrous metals, pure metals and/or nonferrous alloys showing the above surface to volume ratio.

The metal matrix may be provided in any typical form which seems suitable to the person skilled in the art. According to preferred embodiments, the metal matrix is provided in the form of balls, filters, meshes, pins, powders, granulates, swarfs and/or three-dimensional porous or non-porous structures. Metal pins and metal granulates are preferred. In general, metal granulates show a higher surface to volume ratio than metal pins and are therefore especially preferred. Of course, metal materials having a surface to volume ratio similar to metal granulates, are also especially preferred according to the invention.

According to especially preferred embodiments, the metal matrix used is selected from X20Cr13, X30Cr13, X46Cr13, X17CrNi16-2, X90CrMoV18, X40CrMoVN16-2, X5CrNi18-10/X2CrNi19-11, X8CrNiS18-9, X10CrNi18-8, X2CrNiMo18-14-3, X5CrNiCuNb16-4, X3CrNiCuTiNb12-9, X2CrNiMo18-15-3, X4CrNiMnMo21-9-4, Ti CP1, Ti CP4, Ti2, Ti4, TiAl6V4, TiAl6Nb7, K13C20N126Fe15D7, BioDur® 108 Alloy, CoCr28Mo, Custom 455, F-75, Alloy 25, AISI 317L, and/or AISI 431. A person skilled in the art is aware of the respective designations. If not defined otherwise, the designations used are described in Table 1 and Table 2. Table 1 shows preferred metal pin types according to the present invention comprising different designations for the same type of metal pins.

**Table 1: Preferred metal pin types**

| **ID** | **AISI/ASTM/UNS/other** | **DIN** | **WR** |
|---|---|---|---|
| 1 | AISI 420 | X20Cr13 | 1.4021 |
| | AISI 420A | | |
| | ASTM F899 | | |
| 2 | AISI 420B | X30Cr13 | 1.4028 |
| | ASTM F899 | | |
| 3 | AISI 420 | X46Cr13 | 1.4034 |
| | AISI 420C | | |
| | ASTM F899 | | |
| 4 | AISI ~ 431 | X17CrNi16-2 | 1.4057 |
| | ASTM F899 | | |
| 5 | AISI ~ 440B | X90CrMoV18 | 1.4112 |
| 6 | AISI 420 MOD | X40CrMoVN16-2 | 1.4123 |
| | ASTM F899 | | |
| 7 | AISI 304 (V2A)/AISI 304L | X5CrNi18-10/X2CrNi19-11 | 1.4301/1.4306 |
| | ASTM F899 | | |
| 8 | AISI 303 | X8CrNiS18-9 | 1.4305 |
| | ASTM F899 | | |
| 9 | AISI ~ 301/AISI 302 | X10CrNi18-8 | 1.4310 |
| | ASTM F899 | | |
| 10 | AISI 316L | X2CrNiMo18-14-3 | 1.4435 |
| 11 | AISI 630 | X5CrNiCuNb16-4 | 1.4542 |
| | (17-4PH) | | |
| | ASTM F899 | | |
| | UNS S17400 | | |
| 12 | AISI XM-16 | X3CrNiCuTiNb12-9 | 1.4543 |
| | ASTM F899 | | |
| 13 | AISI ~ 316LVM | X2CrNiMo18-15-3 | 1.4441 |
| | ASTM F138 | | |
| | UNS S31673 | | |
| 14 | ASTM F1586 | X4CrNiMnMo21-9-4 | 1.4472 |
| 15 | ASTM B 348 | Ti2 | 3.7035 |
| | ASTM F 67 | | |
| 16 | ASTM B 348 | Ti4 | 3.7065 |
| | ASTM F 67 | | |
| 17 | ASTM B 348 | TiAl6V4 | 3.7165 |
| | ASTM F 136 | | |
| 18 | AISI F1295 | TiAl6Nb7 | 9.9367 |
| 19 | ASTM F1058 | K13C20N126Fe15D7 | 2.4711 |
| | UNS R3003 | | |
| | UNS R3008 | | |
| 20 | UNS S29108 | - | - |
| | BioDur^{®} 108 Alloy | | |
| 21 | ASTM F1537 alloy 1 | CoCr28Mo | - |
| | ASTM F899 | | |
| | UNS R31537 | | |
| | Cobalt BC | | |

| | | | |
|---|---|---|---|
| Abbreviations: ID (identitication number), AISI (American Iron and Steel Institute), ASTM (American Society of Mechanical Engineers), DIN (German Institute for Standardization), UNS (Unified Numbering System), WR (German Material Number), and/or other descriptions. | | | |

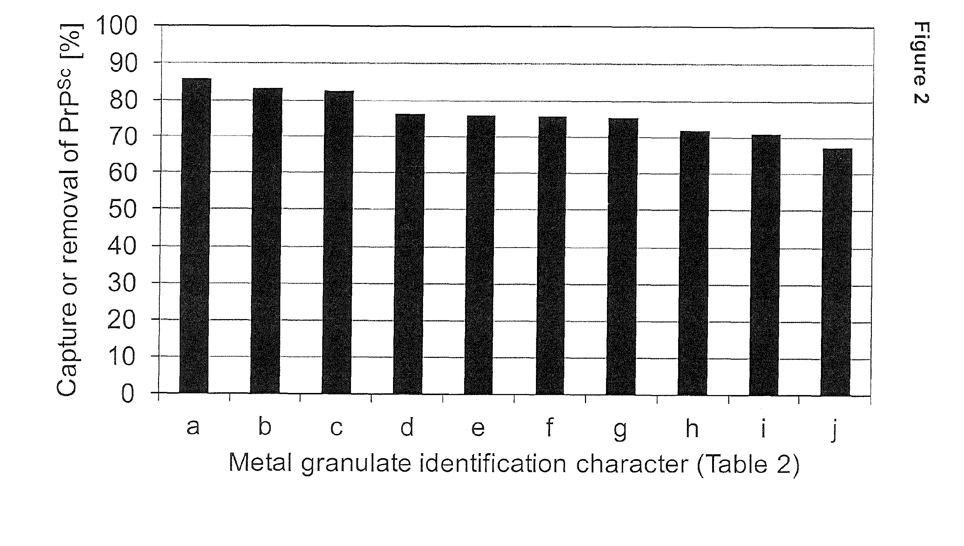

Especially preferred metals used according to the invention are characterized by metal IDs 17 - 21 of Table 1. Surprisingly, these especially preferred metals show a low average protein adsorption/binding but a high selectivity towards PrP^{C} and/or PrP^{Sc} (see Figure 2).

Preferred metal granulate types are shown in Table 2.

**Table 2: Investigated metal granulate types**

| **ID** | **Designation** |
|---|---|
| a | Custom 455 IARM 16B |
| b | F-75 UNS R30075 IARM 208B |
| c | Alloy 25 IARM 96C |
| d | Ti CP1 (Grade 4) UNS R50700 IARM 174C |
| e | 17-4PH UNS S17400 IARM 23C |
| f | AISI 316L IARM-163A |
| g | AISI 317L UNS S31703 IARM 153B |
| h | Ti CP4 (Grade 1) UNS R50250 IARM 312A |
| i | AISI 431 UNS S43100 IARM 12B |
| j | AISI 420 IARM-154A |

| | |
|---|---|
| Abbreviations: ID (identification character), AISI (American Iron and Steel Institute), IARM (Analytical Reference Materials International, Golden, USA), UNS (Unified Numbering System), and/or other descriptions. | |

The target fluid which may be used according to the invention may be any protein-containing solution. In terms of the present invention, "fluid" may comprise inter alia essentially liquid compositions, as well as suspensions, homogenates and/or extracts etc. The target fluid may be highly complex and protein-rich, such as blood plasma, but also samples of lower complexity and/or a low protein concentration may be used. According to a preferred embodiment, the target fluid comprises ≥ 10mg/ml protein, more preferably ≥ 25 mg/ml protein and even more preferably ≥ 50mg/ml protein. The protein-concentration of the target fluid may be even in the range of 60-85 mg/ml such as in the case of blood plasma. The target fluid may be derived from a human sample but, of course, also target fluids from animals are encompassed, in particular bovine target fluids and target fluids from sheep, elk and/or deer.

The target fluid, which is subject to the method according to the present invention, may be selected from body fluids such as blood, serum, plasma, urine, saliva, tears, semen, cerebrospinal fluid, pharmaceutical and medical solutions such as blood derived products, cosmetic liquid formulations, foods such as milk, fluids from agriculture and/or homogenates. According to an especially preferred embodiment, the target fluid is plasma or plasma-derived, in particular human plasma or human plasma-derived. A further example for a target fluid according to the present invention may be any intermediate product and/or solution present in pharmacological, medical, cosmetic, food and/or agricultural processes. Lowering the concentration of amyloidogenic proteins, e.g. pathogenic prions, may also be used *in vitro,* of course, for example in blood and/or plasma samples to be reinfused to a patient.

According to the method of the present invention, detergents may be added to the target fluid, in particular if the metal matrix was not pretreated/coated with detergents before. According to an especially preferred embodiment at least one detergent is present. In terms of the present application, a detergent is any surfactant or a mixture of surfactants having "cleaning properties in dilute solutions." A detergent or surfactant lowers the surface tension of a liquid or the interfacial tension between two liquids.

A detergent according to the present invention may be preferably selected from ethoxylates, cationic detergents, anionic detergents and/or non-ionic or zwitterionic detergents, wherein non-ionic detergents are especially preferred. Non-ionic detergents are characterized by their net uncharged, hydrophilic headgroups. For example, they may be based on polyoxyethylene glycol (e.g. Tween, Triton, Brij etc.), glycosides (e.g. maltosides, octyl-thioglcoside etc.), lipids (e.g. HEGAs), phosphine oxides, bile acids (e.g. DOC) and /or CHAPS. According to an especially preferred embodiment, the present invention relates to nonionic detergents such as Triton x-100, Nonidet P40, CHAPS, and/or IGEPAL CA-630.

Depending on the protein concentration of the sample to be treated the end concentration of the detergent in the sample is preferably from 0.5 to about 5 % (weight/volume; w/v), more preferably 0.75 to 4.5 % (w/v) and even more preferably about 1.0 to 4.0 % (w/v) and most preferably about 1.5 % to 3.5 % (w/v).

A preferred embodiment of the present invention relates to a method comprising a pre-treatment of a metal matrix with at least one detergent as described above. Consequently, a minimized adsorption of non-pathogenic proteins such as PrP^{c} is achieved as, in return, more amyloidogenic proteins, such as pathogenic prions PrP^{Sc}, remain adhered to the metal. In a preferred embodiment, this pre-treatment is performed with a solution comprising 1 to 3% (w/v) detergent, e.g. 2% Triton X-100 at any suitable temperature, in particular at room temperature. The duration of the pre-treatment reaction, is e.g. dependent from the temperature and is normally approx. 60 minutes to 180 minutes, preferably about 120 minutes at room temperature. After the pre-treatment reaction, the pre-treated metal matrix is preferably rinsed with water only briefly. The surface-oriented detergent can be dissolved e.g. by treatment in an ultrasonic bath so that the aimed effect is lost.

According to a preferred embodiment contacting step (a) is carried out at a temperature of about 4°C to about 30°C, more preferably about 10°C to about 25°C. Of course, the inventive method can be carried out at room temperature.

An effective contact time between the target fluid and the metal matrix may be any time sufficient for effective binding of the amyloidogenic protein to be removed, isolated, concentrated etc. The contact time can be determined by the person skilled in the art. Of course, the contact time is dependent from the special purpose to be achieved. The contact time may be for example dependent on the target fluid itself, the volume of the target fluid, the type metal matrix used, the temperature at which the inventive method is carried out, the velocity at which the target fluid is streamed over the metal matrix etc. The contact time may vary between seconds and a couple of hours. The contact time is preferably at least 0.1 min, more preferably at least 5 min, but may also be at least 16 h, depending on how the method according to the present application is applied.

The method according to the present invention can be applied in a continuous manner, such as in a flow-through mode, or in a discontinuous manner, such as in a batch mode. In the flow-through mode, the velocity of the target fluid streaming over the metal matrix can be adjusted by the person skilled in the art to provide optimal results.

The method according to the present invention may comprise a detection and/or analysing step for the detection and/or analysis of the adsorbed and/or captured amyloids, amyloidogenic proteins, and/or proteins with the ability to become amyloidogenic, fragments and/or derivatives thereof. The detection step may be carried out by immunoassay, bioassay, cell assay and/or physical methods such as episcopic differential interference contrast and/or epifluorescence microscopy.

As already outlined, the present invention allows in particular the selective capture of amyloidogenic proteins onto a metallic surface, i.e. metal matrix. Thus, according to an especially preferred embodiment of the present invention amyloidogenic proteins and in particular pathogenic prions such as PrP^{Sc} can selectively be captured, isolated and/or concentrated onto metals if
(i) the protein concentration of the contacting solution is high such as 10 mg/ml, and/or
(ii) detergents are added to the contacting solution, and/or during a detergent pre-treatment and/or detergent coating step.

In this particular embodiment the protein concentration is preferably >10 mg/ml and the concentration of detergent is 0.5 % to 5.0 % (w/v), more preferably 1.0 % to 4.0 % (w/v). Of course, all further ranges specified above with regard to protein concentration and detergent concentration represent further preferred embodiments.

Under these conditions normal prions, i.e. non-pathogenic prions, weakly adsorb onto the metal matrix due to their low capture affinity compared to pathogenic prions. If the used metal or the metal matrix as previously described is pre-treated with detergents, a reduction of the adsorption of proteins, such as PrP^{C} is achieved.

This results in more PrP^{Sc} binding sites and thus higher PrP^{Sc} capture yields, in particular in protein rich solutions. Weakly bound proteins can be removed by subsequent washing and PrP^{Sc} detection thus be simplified.

After binding of the pathogenic prions onto the metal matrix, for example, detection thereof can be carried out for example with standard high affinity prion antibodies. Such prion antibodies are known from the prior art used in TSE diagnostics but are not able to distinguish between pathogenic prions and non-pathogenic prions. Also only a limited number of PrP^{Sc}-specific antibodies has been described, but their affinity appears to be too low for diagnostic applications. This problem is solved by the present invention providing a metal matrix showing an enriched pathogenic prion fraction compared to non-pathogenic prions.

According to another especially preferred embodiment the method according to the present invention may be used for process control and/or screening for the presence of amyloidogenic proteins. According to this embodiment, a device comprising a metal matrix used according to the invention may be contacted with the entire target fluid or relevant part of if instead of withdrawing a small volume segment of a target fluid for the purpose of analysis. Of course, this device may be part of a continuous flow or batch system with and without an additional regulation step through the device. Contacting the target fluid with the device enables capture of amyloidogenic proteins such as PrP^{Sc} on the metal matrix. The capture/removal can be carried out overnight, during transport, in the presence of detergent during SD treatment etc. After incubation the device itself can be analyzed for capture of amyloidogenic proteins. This approach in particular enables quality controls, screening tests for plasma pharmaceutical products or fluids, etc.

In order to carry out step (a) of the inventive method a contacting device may be used. Thus, still a further aspect of the present invention relates to a device
(a) comprising a contacting unit for carrying out the inventive method and optionally
(b) a collection and/or detection unit.

The contacting unit comprises the metal matrix. The contacting device may be combined with a collecting unit and/or detection unit. The detection unit may be, for example, a detection chamber and/or detection well, such as a microtiter plate. The contacting unit, the collection unit and the detection unit may be arranged in any way known to a person skilled in the art.

According to an especially preferred embodiment, the contacting unit, the collection unit and/or detection unit are integrated. For example, the units may be integrated into a flow-through/circling system moving a target fluid possibly containing amyloidogenic proteins such as pathogenic prions. This integration eases handling and improves overall amyloidogenic protein capture and efficiency and safety, providing a method easing less medium transfer steps. In a preferred embodiment the contacting unit, collection unit and/or detection chamber are integrated in a detection unit.

As already outlined above, the inventive method may be carried out in flow-through mode, i.e. the contacting unit can be used during collection and/or detection. The detection unit, e.g. one or several wells in a microtiter plate, may be coated with metal, whereas the metal surface per volume can be enlarged by means of a three-dimensional metal substructure. The metal matrix as outlined above, such as metal swarfs, balls, and/or powder is positioned in the chamber and retained by a lid, filter or due to the magnetic properties.

The device is contacted with a fluid possibly containing amyloidogenic proteins, e.g. pathogenic prions. Alternatively, the device may be integrated into a flow-through/circulating system moving a fluid possibly containing amyloidogenic proteins, e.g. pathogenic proteins, thereby concentrating the amyloids on the metal surface of the device. Subsequent washing steps can be performed directly in the device. In one embodiment the innovative device may remain in a fluid for a prolonged period of time, similar to a dosimeter. This leads to "cumulative" results and not a snapshot analysis of a small volume fluid segment.

For the sake of completeness, it has to be mentioned that besides *in vitro* methods the same principle can be used also *in vivo* in a fluid of a living organism, e.g. blood stream, cardiovascular system, etc. to lower the concentration of amyloidogenic proteins, e.g. pathogenic proteins.

A further aspect relates to the use of at least one metal or alloy selected from the group consisting of for X20Cr13, X30Cr13, X46Cr13, X17CrNi16-2, X90CrMoV18, X40CrMoVN16-2, X5CrNi18-10/X2CrNi19-11, X8CrNiS18-9, X10CrNi18-8, X2CrNiMo18-14-3, X5CrNiCuNb16-4, X3CrNiCuTiNb12-9, X2CrNiMo18-15-3, X4CrNiMnMo21-9-4, Ti CP1, Ti CP4, Ti2, Ti4, TiAl6V4, TiAl6Nb7, K13C20N126Fe15D7, BioDur® 108 Alloy, CoCr28Mo, Custom 455, F-75, Alloy 25, AISI 317L, and/or AISI 431 for isolating, removal, concentrating and/or purifying amyloids, amyloidogenic proteins, and/or proteins with the ability to become amyloidogenic, fragments and/or derivatives thereof. The use of metals and/or alloys 17-21 according to Table 1 is particularly preferred.

The present invention relates further to a method for determining the capture yield and/or capture affinity of a given metal and/or metal matrix for amyloids, amyloidogenic proteins and/or proteins with the ability to become amyloidogenic. In this context "capture yield" describes the absolute or relative amount of a given amyloid, any amyloidic protein and/or protein with the ability to become amyloidogenic and after a pre given incubation time, whereas "capture affinity" relates to a value reached at the thermodynamic equilibrium. This inventive method may comprise the steps pre-treating and/or coating a given metal and/or metal matrix with a detergent as described herein above and/or comparison of captured and/or removed amyloids, amyloidogenic proteins and/or proteins with the ability to become amyloidogenic with a respective reference sample. Such a comparison step may comprise quantifiying PrP^{Sc} and PrP^{C} capture or removal using metal matrixes by comparison with a respective reference sample. An exemplified method is described in Example 1 of the present invention. As already described, in a preferred embodiment, a respective method can be a part of the method according to claim 1 of the invention. But there are other uses for this method as well. For example, the method and/or corresponding test system, those identifying of metal matrixes with low prion affinities, which is suitable for the instruments or devices where minimal contamination with prions is of advantage.

### BRIEF DESCRIPTION OF THE FIGURES AND TABLES

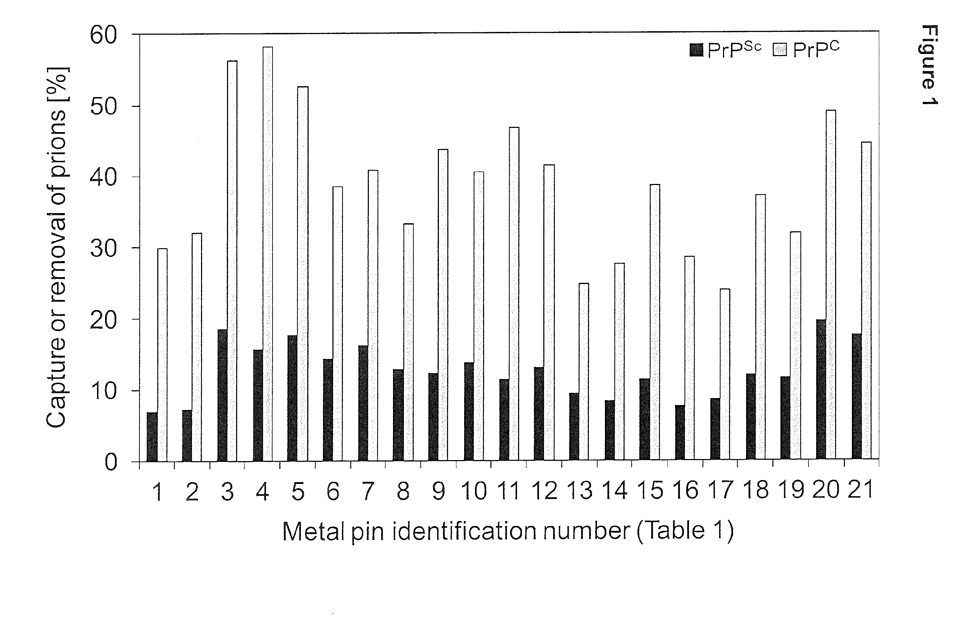

Figure 1 is a graphic representation of the capture or removal of PrP^{Sc} and PrP^{C} from scrapie brain homogenate using surface area standardized metal pins (156 mm²). Metal pin designations are summarized in Table 1. Materials and methods are as indicated in Example 1.
Figure 2 is a graphic representation of the capture or removal of PrP^{Sc} from scrapie brain homogenate using metal granulates. Metal granulate particle size distribution using mesh size is 12 - 32, designations are summarized in Table 2. Materials and methods are as indicated in Example 1.

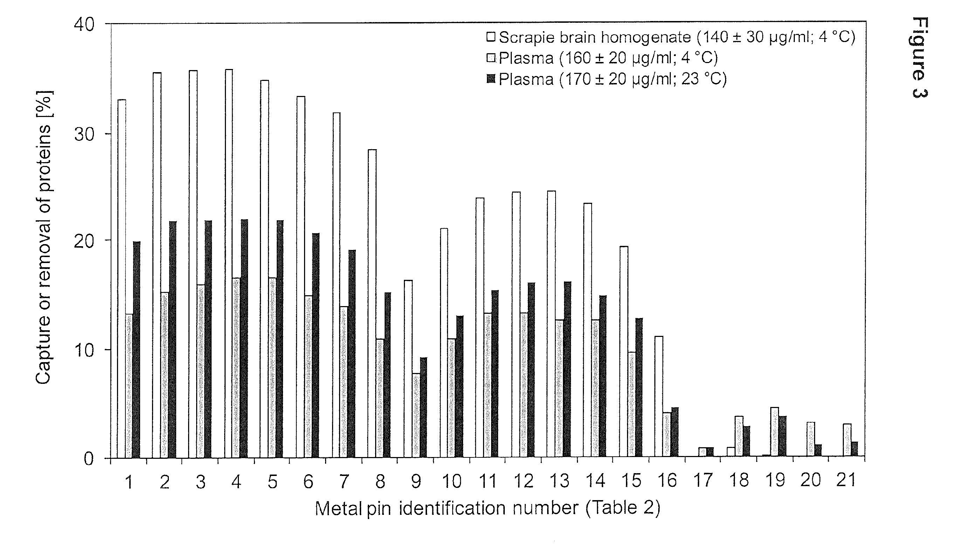

Figure 3 is a graphic representation of the capture or removal of proteins from of scrapie brain homogenate and human plasma using metal pins. Initial protein concentration and temperature during incubation are indicated in brackets. Metal pin designations are summarized in Table 1. See Example 2 for details regarding materials and methods.

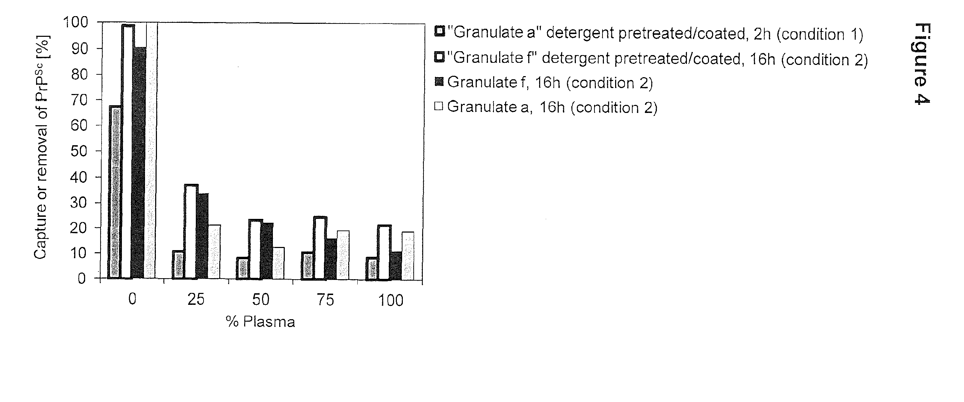

Figure 4 is a graphic representation of the capture or removal of PrP^{Sc} present in human plasma and dilutions thereof using metal granulates (see Table 2). X-Axis indicates the volume fraction of plasma in PBS, 100 % refers to undiluted plasma, 0 % refers to PBS. The solutions are supplemented with Triton X-100 (condition 1), or a combination of CHAPS, benzonase, and protease inhibitors (condition 2). Materials and methods are as indicated in Example 3.

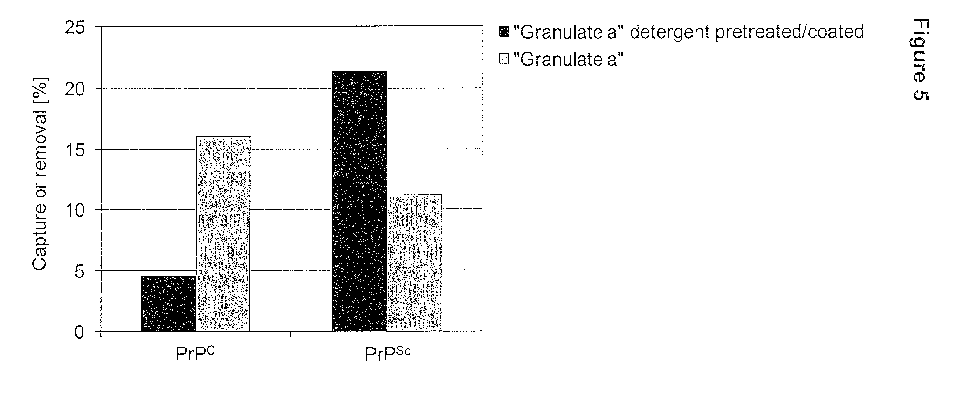

Figure 5 shows the influence of metal matrix pretreatment/coating with detergent on the capture or removal of PrP^{Sc} and PrP^{C} in human plasma. Utilized metal matrix: "granulate a" (see Table 2), employed detergent during pretreatment/coating: Triton X-100. See Example 4 for details regarding materials and methods.

### Examples

### Example 1: Capture or removal of PrP^{Sc} and PrP^{C} from scrapie brain homogenate using metal matrices

### Preparation:

Deionized and filtered water was used for the preparation of solutions. Phosphate buffered saline (PBS, pH 7.2) was additionally filtered by means of Whatman cellulose acetate membrane filters (0.45 µm pore size).

Metal matrices (*Pins:* Klein SA, Biel, Switzerland; 156 mm² standardized surface area; see Table 1. *Granulates:* LGC Standards GmbH, Wesel, Germany; standardized particle size distribution using mesh size: 12 - 32; see Table 2) were cleaned and/or sterilized, if necessary, with methanol (25 °C; continuous shaking), acetone (25 °C; ultrasonication), 0.375 M sodium hydroxide (70 °C; ultrasonication), 2 % Triton X-100 (25 °C; ultrasonication), or a the successive application of Dr. Weigert neodisher LaboClean FLA (85 °C), Dr. Weigert neodisher Z (25 °C, 50 °C), Dr. Weigert neodisher LaboClean FLA (75 °C), Dr. Weigert neodisher Z (25 °C) in a washing machine, followed by several ultrasonication steps in water, and drying at 45 °C. The metal matrices were allowed to cool to room temperature before any capture or removal procedure was initiated. 10 % scrapie brain homogenate was prepared using hamster brains infected with the 263K hamster-adapted agent.

### Procedure:

*Metal pins:* The 10 % scrapie brain homogenate was centrifuged at 4500 x g for 30 min (4 °C). The clarified supernatant solution containing PrP^{Sc} and PrP^{C} was 100-fold diluted in PBS (4 °C). 500 µl of this solution was added to every metal pin in a tube (0.5 ml Eppendorf Protein LoBind Tube). The tubes were closed and the mixtures were incubated for 2 h at 4 °C on an overhead shaker (20 rpm). Reference sample: 500 µl of the same 100-fold diluted stock solution containing PrP^{Sc} and PrP^{C} was transferred into an empty 0.5 ml tube (without metal pin). The tube was closed and incubated for 2 h at 4 °C on an overhead shaker (20 rpm). Subsequently, supernatants were withdrawn and directly analyzed for the capture or removal of prions.

*Metal granulates:* The 10 % scrapie brain homogenate was centrifuged at 4500 x g for 30 min (4 °C), following filtration of the obtained supernatant using 0.45 µm NANOSEP MF centrifugal filters (10'000 x g, 4 °C), following filtration of the flow-through using 0.2 µm NANOSEP MF centrifugal filters (10'000 x g, 4 °C). The highly clarified solution containing PrP^{Sc} and PrP^{C} was 50-fold diluted in PBS (4 °C). 1000 µl of this solution was added to 0.5 cm³ of every metal granulate in a tube (1.5 ml Eppendorf Protein LoBind Tube). The tubes were closed and the mixtures were incubated for 2 h at 4 °C on an overhead shaker (30 rpm). Reference sample: 1000 µl of the same 50-fold diluted solution containing PrP^{Sc} and PrP^{C} was transferred into an empty 1.5 ml tube (without metal granulate). The tube was closed and incubated for 2 h at 4 °C on an overhead shaker (30 rpm). Subsequently, supernatants were withdrawn and directly analyzed for the capture or removal of prions.

### Analysis:

A conformation-dependent immunoassay (CDI) was applied to differentiate between total PrP and PrP^{C} concentrations. 20 µl of supernatant solution was either supplemented with 20 µl PBS or 20 µl guanidine hydrochloride solution (8 M). Guanidine hydrochloride supplemented samples were incubated for 5 min at 80 °C. The samples were allowed to cool to room temperature. Subsequently, HPE dilution buffer (High Performance ELISA, Sanquin) was added to both PBS and guanidine hydrochloride supplemented samples. Analysis was carried out using a sandwich enzyme-linked immunosorbent assay (ELISA). For sandwich ELISA, white 96 well flat-bottom plates (Nunc MaxiSorp) were prepared with 3F4 as a capture antibody (5 µg/ml in carbonate-bicarbonate buffer pH 9.4), and blocked with SuperBlock Blocking Buffer (Thermo Scientific). Plasma samples were diluted in HPE dilution buffer and applied to the plate. Biotinylated SAF32 antibody (400 ng/ml in HPE) was added, following streptavidin-poly-HRP (Thermo Scientific; in HPE), and luminescent detection using SuperSignal Femto solution (Thermo Scientific). Plate washing between the different steps, except after blocking, was carried out with tris buffered saline (TBS; pH 7.2) supplemented with 0.05 % Tween-20. PrP^{Sc} and PrP^{C} capture or removal using metal matrices was quantified by comparison with the respective reference sample.

### Results:

Surprisingly it was found out that metals in general, such as high-alloyed steel, low-alloyed steel, nonferrous metals, but also nonferrous alloys, are able to capture/remove PrP^{Sc} and PrP^{C} (Figure 1 - 2). Interestingly, PrP^{Sc} or PrP^{C} capture/removal yields can differ by a factor of two to three, depending on the metal (Figure 1). Comparison of metal pins (Figure 1) and metal granulates (Figure 2) shows that prion capture/removal yields can be increased with the use of surface area optimized metal matrices, i.e. metal matrices having a high surface to volume ratio. For the employed metal pins a surface to volume ratio of around 12 cm²/cm³ is calculated. The surface to volume ratio for the metal granulates (> 18 cm² depending on the metal, per 0.5 cm³) is at least three times larger, explaining the higher PrP^{Sc} capture/removal yield (measured initial PrP^{Sc} concentrations of both metal pin and metal granulate reference samples were identical).

### Example 2: Capture or removal of proteins from scrapie brain homogenate and human plasma using metal pins

### Preparation:

Deionized and filtered water was used for the preparation of solutions. Phosphate buffered saline (PBS, pH 7.2) was additionally filtered by means of Whatman cellulose acetate membrane filters (0.45 µm pore size). Metal pins (Klein SA, Biel, Switzerland; 156 mm² standardized surface area; see Table 1) were cleaned and/or sterilized, if necessary, with methanol (25 °C; continuous shaking), acetone (25 °C; ultrasonication), 0.375 M sodium hydroxide (70 °C; ultrasonication), 2 % Triton X-100 (25 °C; ultrasonication), or a the successive application of Dr. Weigert neodisher LaboClean FLA (85 °C), Dr. Weigert neodisher Z (25 °C, 50 °C), Dr. Weigert neodisher LaboClean FLA (75 °C), Dr. Weigert neodisher Z (25 °C) in a washing machine, followed by several ultrasonication steps in water, and drying at 45 °C. The metal matrices were allowed to cool to room temperature before any capture or removal procedure was initiated. 10 % scrapie brain homogenate was prepared using hamster brains infected with the 263K hamster-adapted agent.

### Procedure:

*Scrapie brain homogenate:* The 10 % scrapie brain homogenate was centrifuged at 4500 x g for 30 min (4 °C). The clarified supernatant solution containing PrP^{Sc} and PrP^{C} was 100-fold diluted in PBS (4 °C). 500 µl of this solution was added to every metal pin in a tube (0.5 ml Eppendorf Protein LoBind Tube). The tubes were closed and the mixtures were incubated for 2 h at 4 °C on an overhead shaker (20 rpm). Reference sample: 500 µl of the same 100-fold diluted stock solution containing PrP^{Sc} and PrP^{C} was transferred into an empty 0.5 ml tube (without metal pin). The tube was closed and incubated for 2 h at 4 °C on an overhead shaker (20 rpm). Subsequently, samples were withdrawn and directly analyzed for the removal or capture of proteins.

*Plasma:* Human fresh frozen plasma was diluted with PBS to obtain a similar initial protein concentration as the scrapie brain homogenate solution added to every metal pin. 500 µl of this plasma solution (4 °C or 23 °C) was added to every metal pin in a tube (0.5 ml Eppendorf Protein LoBind Tube). The tubes were closed and the mixtures were incubated for 2 h at 4 °C or 23 °C on an overhead shaker (20 rpm). Reference sample: 500 µl of the same plasma solution was transferred into an empty 0.5 ml tube (without metal pin). The tube was closed and incubated for 2 h at 4 °C or 23 °C on an overhead shaker (20 rpm). Subsequently, samples were withdrawn and directly analyzed for the removal or capture of proteins.

### Analysis:

The Pierce BCA Protein Assay Kit (Thermo Fisher Scientific Inc, Rockford, USA) was used for the quantification of total protein. Protein capture/removal using metal matrices was quantified by comparison with the respective reference sample.

### Results:

The employed metal matrices show a surprisingly similar capture/removal pattern for proteins from both scrapie brain homogenate and human plasma under the investigated conditions (Figure 3). Most surprisingly, pins 17 - 21 capture/remove exceptionally few scrapie brain homogenate and plasma proteins, while at the same time exhibiting a prion capture/removal performance similar to other metals (Figure 1). The high capture or removal selectivity of materials based on pins 17 - 21 towards prion proteins can be useful for:
(i) diagnostic applications, where prion detection is carried out directly on the metal matrix and the amount of analysis interfering, co-captured substances such as proteins needs to be minimal.
(ii) production processes, where preventive removal of prions is necessary, but the co-removal of target proteins/substances needs to be minimal for economical reasons.

### Example 3: Capture or removal of PrP^{Sc} present in human plasma and dilutions thereof using metal granulates

### Preparation:

Deionized and filtered water was used for the preparation of solutions. Phosphate buffered saline (PBS, pH 7.2) was additionally filtered by means of Whatman cellulose acetate membrane filters (0.45 µm pore size). Detergent pretreatment/coating: 1 gram of metal "granulate a" or "granulate f" (LGC Standards GmbH, Wesel, Germany; standardized particle size distribution using mesh size: 12 - 32; see Table 2) was incubated with 4 ml of 2 % Triton X-100 on an overhead shaker at 5 rpm for 2 h (room temperature). Next, the solution was removed and the granulates were rinsed three times with water (5 ml/g). The metal matrices were dried at room temperature before any capture or removal procedure was initiated. 10 % Scrapie brain homogenate was prepared using hamster brains infected with the 263K hamster-adapted agent. Prior to use, the homogenate was highly clarified according to the following procedure: centrifugation at 4500 x g for 30 min (4 °C), following filtration of the obtained supernatant using 0.45 µm NANOSEP MF centrifugal filters (10'000 x g, 4 °C), following filtration of the flow-through using 0.2 µm NANOSEP MF centrifugal filters (10'000 x g, 4 °C).

### Proced ure:

The clarified solution containing PrP^{Sc} and PrP^{C} was 47-fold diluted in human fresh frozen plasma (designated as 100 % Plasma), PBS (designated as 0 % Plasma), or mixtures thereof (25, 50, and 75 % plasma volume fraction). Condition 1: 1551 µl of every solution was supplemented with 41.3 µl of 40 % Triton X-100 (w/v) and 58.7 µl of PBS. Condition 2: 1551 µl of every solution was supplemented with either 1 µl of benzonase (41.3 U), 16.5 µl of protease inhibitor cocktail (104 mM AEBSF, 80 µM Aprotinin, 4 mM Bestatin, 1.4 mM E-64, 2 mM Leupeptin, 1.5mM Pepstatin A, in DMSO), and 82.5 µl of 40 % CHAPS (w/v), as described in literature (Edgeworth et al (2011) Lancet 377: 487-493). Every mixture was incubated for 10 min at 23 °C. Subsequently, 0.75 ml was transferred into a tube (1.5 ml Eppendorf Protein LoBind Tube) containing 0.5 cm³ of metal granulate, and 0.75 ml was transferred into an empty tube (reference sample). The tubes were closed and the mixtures were incubated for 2 h or 16 h at 23 °C on an overhead shaker (30 rpm). After incubation, samples were withdrawn and directly analyzed for the removal or capture of prions.

### Analysis:

A conformation-dependent immunoassay (CDI) was applied to differentiate between total PrP and PrP^{C} concentrations. 20 µl of supernatant solution was either supplemented with 20 µl PBS or 20 µl guanidine hydrochloride solution (8 M). Guanidine hydrochloride supplemented samples were incubated for 5 min at 80 °C. The samples were allowed to cool to room temperature and then diluted drop-wise into HPE dilution buffer (High Performance ELISA, Sanquin) under continuous mixing. The same dilution in HPE was carried out for PBS supplemented samples. Analysis was carried out using a sandwich enzyme-linked immunosorbent assay (ELISA). For sandwich ELISA, white 96 well flat-bottom plates (Nunc MaxiSorp) were prepared with 3F4 as a capture antibody (2 µg/ml in carbonate-bicarbonate buffer pH 9.4), and blocked with SuperBlock Blocking Buffer (Thermo Scientific). Plasma samples were diluted in HPE dilution buffer and applied to the plate. Biotinylated SAF32 antibody (135 ng/ml in HPE) was added, following streptavidin-poly-HRP (Thermo Scientific; in HPE), and luminescent detection using SuperSignal Femto solution (Thermo Scientific). Plate washing between the different steps, except after blocking, was carried out with tris buffered saline (TBS; pH 7.2) supplemented with 0.05 % Tween-20. PrP^{Sc} and/or PrP^{C} capture or removal using metal granulate was quantified by comparison with the respective reference sample.

### Results:

In general, PrP^{Sc} capture or removal yields decrease with increasing plasma protein concentration, indicating that sample dilution prior to contacting with a metal matrix could improve capture or removal yields. PrP^{Sc} capture is dependent on contact time: capture yields after 16 h are higher than after 2 h for all plasma dilutions and conditions investigated (Figure 4). Surprisingly it was found out that metal matrix pretreatment/coating with detergent leads to improved PrP^{Sc} capture or removal yields, as demonstrated for "granulate f".

### Example 4: Influence of metal matrix pretreatment/coating with detergent on the capture or removal of PrP^{Sc} and PrP^{C} in human plasma

### Preparation:

Solutions, detergent pretreatment/coating, and 10 % scrapie brain homogenate was prepared as described in Example 3.

### Procedure:

The clarified solution containing PrP^{Sc} and PrP^{C} was 47-fold diluted in human fresh frozen plasma. 1551 µl of this solution was supplemented with 1 µl of benzonase (41.3 U), 16.5 µl of protease inhibitor cocktail (104 mM AEBSF, 80 µM Aprotinin, 4 mM Bestatin, 1.4 mM E-64, 2 mM Leupeptin, 1.5mM Pepstatin A, in DMSO), and 82.5 µl of 40 % CHAPS (w/v), as described in literature (Edgeworth et al (2011) Lancet 377: 487-493). The mixture was incubated for 10 min at 23 °C. Subsequently, 0.75 ml was transferred into a tube (1.5 ml Eppendorf Protein LoBind Tube) containing 0.5 cm³ of metal "granulate a", and 0.75 ml was transferred into an empty tube (reference sample). The tubes were closed and the mixtures were incubated for 16 h at 23 °C on an overhead shaker (30 rpm). After incubation, supernatants were withdrawn and directly analyzed for the removal or capture of prions.

### Analysis:

Analysis was carried out as described in Example 3.

### Results:

The surprising ability of certain metals to capture prions at higher yields than other proteins is shown in Figure 1 and Figure 3.

Most surprisingly, it was also found that detergent pretreatment/coating of the metal matrix alters prion capture yield in favor of pathogenic prions. In human plasma, "granulate a" pretreatment with Triton X-100 reduced PrP^{C} capture yield by 70 %, while at the same time doubled the amount of captured PrP^{Sc} (Figure 5). Apparently surface coating with detergent reduces metal-interaction with proteins other than PrP^{Sc}, which in turn increases both accessible metal surface area and PrP^{Sc} capture yield.

In summary, metal matrix pretreatment/coating with a suitable detergent can be useful for:
(i) diagnostic applications in complex solutions such as plasma, where the detection of pathogenic prions is carried out directly on the metal matrix and the amount of analysis interfering, co-captured proteins, i.e. cellular prion proteins, needs to be minimal, thereby circumventing the need for PrP^{Sc} specific antibodies and/or PrP^{C} discriminating steps such as e.g. proteolytic processing.
(ii) production processes in complex solutions such as plasma, where preventive removal of pathogenic prions is necessary, but the co-removal of target proteins/substances needs to be minimal for economical reasons.

## Claims

1. Method for isolating, removing, concentrating and/or purifying amyloids, amyloidogenic proteins and/or proteins with the ability to become amyloidogenic, fragments and/or derivatives thereof, comprising a step (a) contacting a metal matrix having a surface to volume ratio between 1 cm²/cm³ and 700 cm²/cm³ with a target fluid under conditions allowing for adsorption and/or capture of present amyloidogenic proteins and/or proteins with the ability to become amyloidogenic, fragments and/or derivatives thereof, wherein at least one detergent may be present during the contacting step (a).

2. The method according to claim 1, wherein the amyloids, amyloidogenic proteins and/or proteins with the ability to become amyloidogenic, fragments and/or derivatives thereof are selected from beta amyloid, IAPP amylin, alpha-synuclein and PrP protein, in particular PrP^{Sc}, PrP^{C}, PrPres and/or recombinant prion protein.

3. The method according to any of claims 1 or 2,
wherein the metal matrix is selected from metal-based materials comprising ferrous material, steel (unalloyed, low-alloyed, high-alloyed), cast iron, nonferrous metals, pure metals, nonferrous alloys.

4. The method according to any of claims 1 to 3,
wherein the metal matrix is provided in the form of balls, filter, meshes, pins, powder, granulate, swarfs and/or three-dimensional porous structures.

5. The method according to any of claims 1 to 4,
wherein the metal matrix has a surface to volume ratio between 10 cm²/cm³ and 500 cm²/cm³.

6. The method according to any of claims 1 to 5,
wherein the metal matrix is selected from X20Cr13, X30Cr13, X46Cr13, X17CrNi16-2, X90CrMoV18, X40CrMoVN16-2, X5CrNi18-10/X2CrNi19-11, XBCrNiS18-9, X10CrNi18-8, X2CrNiMo18-14-3, X5CrNiCuNb16-4,
X3CrNiCuTiNb12-9, X2CrNiMo18-15-3, X4CrNiMnMo21-9-4, Ti CP1, Ti CP4, Ti2, Ti4, TiAl6V4, TiAl6Nb7, K13C20N126Fe15D7, BioDur® 108 Alloy, CoCr28Mo, Custom 455, F-75, Alloy 25, AISI 317L, and/orAISI 431.

7. The method according to any of claims 1 to 6,
wherein the target fluid is selected from body fluids such as blood, serum, plasma, urine, saliva, tears, semen, cerebrospinal fluid, pharmaceutical and medical solutions such as blood derived products, cosmetic liquid formulations, foods such as milk, fluids from agriculture and/or homogenates.

8. The method according to any of claims 1 to 7,
wherein the detergent is present in a concentration of about 0.5% (w/v) to about 5 % (w/v), more preferably about 1 % (w/v) to about 4 % (w/v).

9. The method according to any of claims 1 to 8, comprising a pre-treatment step of the metal matrix with detergent before step (a).

10. The method according to any of claims 1 to 9,
comprising a detection and/or analyzing step for the detection and/or analysis of the adsorbed and/or captured amyloids, amyloidogenic proteins and/or proteins with the ability to become amyloidogenic, fragments and/or derivatives thereof.

11. The method according to claim 10,
wherein the detection step is done by immunoassay, bioassay, cell assay, and/or physical methods such as episcopic differential interference contrast and/or epifluorescence microscopy.

12. The method according to any of claims 10 or 11,
wherein a contacting device being used for carrying out step (a) and a detection device being used for carrying out the detection are integrated.

13. Method for the selective capture of amyloids, in particular PrP^{Sc}, onto a metallic matrix comprising the method of any of claims 1 to 12,
wherein
(i) the protein concentration of the contacting solution is high such as > 10 mg/ml, and/or
(ii) detergents are added to the contacting solution and/or the metallic matrix is pre-treated and/or coated with detergent.

14. Use of a metal or alloy selected from the group consisting of X20Cr13, X30Cr13, X46Cr13, X17CrNi16-2, X90CrMoV18, X40CrMoVN16-2, XSCrNi18-10/X2CrNi19-11, X8CrNiS18-9, X10CrNi18-8, X2CrNiMo18-14-3, X5CrNiCuNb16-4, X3CrNiCuTiNb12-9, X2CrNiMo18-15-3, X4CrNiMnMo21-9-4, Ti CP1, Ti CP4, Ti2, Ti4, TiAl6V4, TiAl6Nb7, K13C20N126Fe15D7, BioDur® 108 Alloy, CoCr28Mo, Custom 455, F-75, Alloy 25, AISI 317L, and/or AISI 431 for isolating, removal, concentrating and/or purifying amyloids, amyloidogenic proteins and/or proteins with the ability to become amyloidogenic, fragments and/or derivatives thereof.

15. Device comprising
(a) a contacting unit for carrying out the method according to any of claims 1-12 and optionally
(b) a collection unit and/or detection unit.

16. A method for determining the capture yield and/or capture affinity of a metal and/or metal matrix for amyloids, amyloidogenic proteins and/or proteins with the ability to become amyloidogenic.
